(19) Europäisches Patentamt / European Patent Office / Office européen des brevets

(11) **EP 2 718 413 B1**

(12) **EUROPEAN PATENT SPECIFICATION**

(45) Date of publication and mention of the grant of the patent:
**15.11.2017 Bulletin 2017/46**

(21) Application number: **12728345.5**

(22) Date of filing: **08.06.2012**

(51) Int Cl.:
*A61K 8/60* (2006.01)  *A61Q 19/10* (2006.01)
*A61K 8/03* (2006.01)  *A61K 8/19* (2006.01)
*C11D 1/94* (2006.01)  *C11D 3/22* (2006.01)

(86) International application number:
**PCT/US2012/041499**

(87) International publication number:
**WO 2012/170784 (13.12.2012 Gazette 2012/50)**

(54) **PERSONAL CARE COMPOSITIONS**

KÖRPERPFLEGEZUSAMMENSETZUNGEN

COMPOSITIONS DE SOINS PERSONNELS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.06.2011 US 201161495543 P**

(43) Date of publication of application:
**16.04.2014 Bulletin 2014/16**

(73) Proprietor: **The Procter & Gamble Company**
**Cincinnati, OH 45202 (US)**

(72) Inventors:
• **WEI, Karl, Shiqing**
  **Mason, Ohio 45040 (US)**
• **STELLA, Qing**
  **Cincinnati, Ohio 45242 (US)**
• **ARREDONDO, Victor, Manuel**
  **West Chester, Ohio 45069 (US)**

• **JI, Wei**
  **Cincinnati, Ohio 45249 (US)**
• **BELCHER, William, Randall**
  **Bellbrook, Ohio 45305 (US)**
• **TIREY, Debra, Ann**
  **Maineville, Ohio 45039 (US)**
• **MENON, Elton, Luis**
  **Mason, Ohio 45040 (US)**

(74) Representative: **Kremer, Véronique Marie Joséphine et al**
**Procter & Gamble Service GmbH**
**IP Department**
**Frankfurter Strasse 145**
**61476 Kronberg im Taunus (DE)**

(56) References cited:
**WO-A2-2009/107062    US-A1- 2011 045 039**
**US-A1- 2011 117 225**

**Description**

FIELD

This application is directed to personal care compositions which include sucrose polyesters as a benefit agent and methods relating thereto.

BACKGROUND

Cleansing the skin is an activity that has been done for millennia. Over time, skin cleansing and related methods for cleansing skin have involved the utilization of soap, surfactants, and the like. Today, one prevalent form of skin cleansing compositions is the liquid form, often known as body wash. Users of body washes enjoy the conveniences these compositions offer; however, the experience is not ideal. As the compositions for cleaning skin have evolved, solutions for the problems associated with these compositions have not. Many of the issues associated with current compositions and methods for skin cleansing, particularly body wash compositions, have not been addressed, and remain issues for users of these products today.

There is, therefore, a need for a personal care composition that provides superior cleaning without the negative elements associated with body washes in the past, including high surfactant concentrations, harshness, stability issues, skin-feel issues, and compatibility issues.

SUMMARY

A personal care composition comprises at least a structured cleansing phase and a benefit phase. The structured cleansing phase comprises: a) an aqueous structured surfactant phase comprising from about 5% to about 20%, by weight of said personal care composition, of an anionic surfactant; b) an amphoteric surfactant, a zwitterionic surfactant, or a combination thereof; and c) a structuring system comprising an electrolyte. The benefit phase comprises from 0.1% to about 50%, by weight of said personal care composition, of a benefit agent comprising a sucrose polyester, wherein the sucrose polyester has an iodine value of 3 or more.

A personal care composition, comprising : a) a structured aqueous phase comprising from about 5% to about 20% of, by weight of the personal care composition, a first surfactant; an amphoteric surfactant, zwitterionic surfactant, or combination thereof; and a structuring system comprising (i) a non-ionic emulsifier, (ii) an associative polymer, and an electrolyte; and b) a benefit phase comprising from about 0.1% to about 50%, by weight of the personal care composition, of a benefit agent comprising a sucrose polyester with an iodine value of about 10 to about 140.

BRIEF DESCRIPTION OF THE DRAWINGS

Fig. 1 is a graph of the dissolution of STnS series compositions;
Fig. 2 is a graph of the rheology profile of the STnS series compositions;
Fig. 3 is a graph of Young's Modulus for the STnS series compositions;
Fig. 4 is a graph capturing the highest dilution maintaining 100% lamellar volume;
Fig. 5 is a graph of the phase transition during dilution of the STnS series compositions;
Fig. 6 is a graph of the lamellar phase volume during dilution level of an ST2S composition with differing cosurfactants;
Fig. 7 is a graph of the rheology profile of STnS compositions with differing associative polymers;
Fig. 8 is a graph of the DPD Curvature of the STnS series compositions;
Fig. 9 is an illustration for determining the third-phase volume;
Fig. 10 depicts a range of esterfication and saturation of a sucrose polyester that can be used in compositions disclosed herein.
Fig. 11 is a graph showing the relationship between the iodine values of different sucrose polyesters and the friction force.

DETAILED DESCRIPTION

Definitions

The devices, apparatuses, methods, components, and/or compositions of the present invention can include, consist essentially of, or consist of, the components of the present invention as well as other ingredients described herein. As used herein, "consisting essentially of" means that the devices, apparatuses, methods, components, and/or compositions may include additional ingredients, but only if the additional ingredients do not materially alter the basic and novel characteristics of the claimed devices, apparatuses, methods, components, and/or compositions.

All percentages and ratios used herein are by weight of the total composition and all measurements made are at 25°C, unless otherwise designated.

All measurements used herein are in metric units unless otherwise specified.

The term "anhydrous" as used herein, unless otherwise specified, refers to those compositions or materials containing less than about 10%, more preferably less than about 5%, even more preferably less than about 3%, even more preferably zero percent, by weight of water.

The term "multiphase" as used herein means that compositions comprise at least two phases which are chemically distinct (e.g. a surfactant phase and a benefit phase). Such phases are in direct physical contact with one another and are not separated by a barrier. The personal care composition can be a multiphase personal care composition where the phases of the personal care composition are blended or mixed to a significant degree. The personal care composition can also be a multiphase personal care composition where the phases of the personal care composition are made to occupy separate but distinct physical spaces inside the package in which they are stored, but are in direct contact with one another (i.e., they are not separated by a barrier and they are not emulsified or mixed to any significant degree).

The term "package" includes any suitable container for a personal care compositions exhibiting a viscosity from about 1,500 centipoise (cP) to about 1,000,000 cP, including but not limited to bottle, tottle, tube, jar, non-aerosol pump and mixtures thereof.

The term "personal care composition" as used herein, refers to compositions intended for topical application to the skin and/or hair. The compositions of the present invention are rinse-off formulations, in which the product is applied topically to the skin or hair and then is subsequently rinsed within seconds to minutes from the skin and/or hair with water, or otherwise wiped off using a substrate. The compositions also may be used as shaving aids. The personal care composition of the present invention is typically extrudable or dispensible from a package. The multiphase personal care compositions typically exhibit a viscosity of from about 1,500 centipoise (cP) to about 1,000,000 cP, as measured by as measured by the Viscosity Method as described in the commonly owned, patent application published on Nov. 11, 2004 under U.S. Publication No. 2004/0223991A1 entitled "Multi-phase Personal Care Compositions" filed on May 7, 2004 by Wei, et al. The multiphase personal care compositions of the present invention can be in the form of liquid, semi-liquid, cream, lotion or gel. Examples of personal care compositions of the present invention can include but are not limited to shampoo, conditioning shampoo, body wash, moisturizing body wash, shower gels, skin cleansers, cleansing milks, hair and body wash, in shower body moisturizer, pet shampoo, shaving preparations, and cleansing compositions used in conjunction with a disposable cleansing cloth.

The phrase "substantially free of" as used herein, unless otherwise specified means that the composition comprises less than about 5%, preferably less than about 3%, more preferably less than about 1% and most preferably less than about 0.1% of the stated ingredient. The term "free of" as used herein means that the composition comprise 0% of the stated ingredient that is the ingredient has not been added to the composition, however, these ingredients may incidentally form as a byproduct or a reaction product of the other components of the composition.

The term "stable," as used herein, means that the multiphase personal care composition comprises less than 10% "third-phase" volume, more preferably less than 5% "third-phase" volume, most preferably less than 1% "third-phase" volume after undergoing the rapid protocol aging and third phase measurement as described below in the "Third-Phase" Method.

The term "structured," as used herein means having a rheology that confers stability on the multiphase composition. The degree of structure is determined by characteristics determined by one or more of the following methods: the Young's Modulus Method, Yield Stress Method, or the Zero Shear Viscosity Method or by the Ultracentrifugation Method, all in the Test Methods below. Accordingly, a surfactant phase of the multiphase composition of the present invention is considered "structured," if the surfactant phase has one or more of the following properties described below according to the Young's Modulus Method, Yield Stress Method, or the Zero Shear Viscosity Method or by the Ultracentrifugation Method. A surfactant phase is considered to be structured, if the phase has one or more of the following characteristics:

A. a Zero Shear Viscosity of at least about 100 Pascal-seconds (Pa-s), at least about 200 Pa-s, at least about 500 Pa-s, at least about 1,000 Pa-s, at least about 1,500 Pa-s, or at least about 2,000 Pa-s; or

B. a Structured Domain Volume Ratio as measured by the Ultracentrifugation Method described hereafter, of greater than about 40%, preferably at least about 45%, more preferably at least about 50%, more preferably at least about 55%, more preferably at least about 60%, more preferably at least about 65%, more preferably at least about 70%, more preferably at least about 75%, more preferably at least about 80%, even more preferably at least about 85%; or most preferably at least about 90%.

C. A Young's Modulus of greater than about 2 Pascal (Pa), more preferably greater than about 10 Pa, even more preferably greater than about 20 Pa, still more preferably greater than about 30 Pa, 40 Pa, 50 Pa, 75 Pa, most preferably greater than 100 Pa.

The term "surfactant component" as used herein means the total of all anionic, nonionic, amphoteric, zwitterionic and cationic surfactants in a phase. When calculations are based on the surfactant component, water and electrolyte are excluded from the calculations involving the surfactant component, since surfactants as manufactured typically are diluted and neutralized.

The term "STnS" as used herein, means sodium trideceth sulfate, where n is defined as the average number of moles of ethoxylate per molecule. Trideceth is a 13 carbon branched ethoxylated hydrocarbon which can comprise an average of at least 1 methyl branch per molecule.

The term "SLS" as used herein, means sodium lauryl sulfate.

The term "visually distinct" as used herein, refers to a region of the multiphase personal care composition having one average composition, as distinct from another region having a different average composition, wherein the regions are visible to the unaided naked eye. This would not preclude the distinct regions from comprising two similar phases where one phase could comprise pigments, dyes, particles, and various additional ingredients, hence a region of a different average composition. A phase generally occupies a space or spaces having dimensions larger than the colloidal or sub-colloidal components it comprises. A phase can also be constituted or re-constituted, collected, or separated into a bulk phase in order to observe its properties, e.g., by centrifugation, filtration or the like.

COMPOSITION

Rinse-off personal care composition come in many forms, like the surfaces they are used upon. For example, rinse-off personal care compositions can be used on skin. Depending on, for example, the care a person takes of their skin, the weather, and overall general health, a person's skin can be anywhere from dry to oily. Utilizing a personal care composition with a cleansing phase can further exacerbate already dry skin or can dry out normal to oily skin. One way to combat the drying effect of surfactants is to include a benefit agent in the composition. Benefit agents can be deposited on the skin during the use of a personal care composition and can act as a replacement or supplemental barrier to the skin to reduce the dry feel left by some surfactants.

The down side to using a benefit agent in a personal care composition is that it can leave the skin feeling oily and/or sticky. Like a drying sensation from a surfactant, the negative sensations from a benefit agent can also cause consumers to not want to use the product. Thus, to develop a successful product, a formulator will need to understand the delicate balance between these issues.

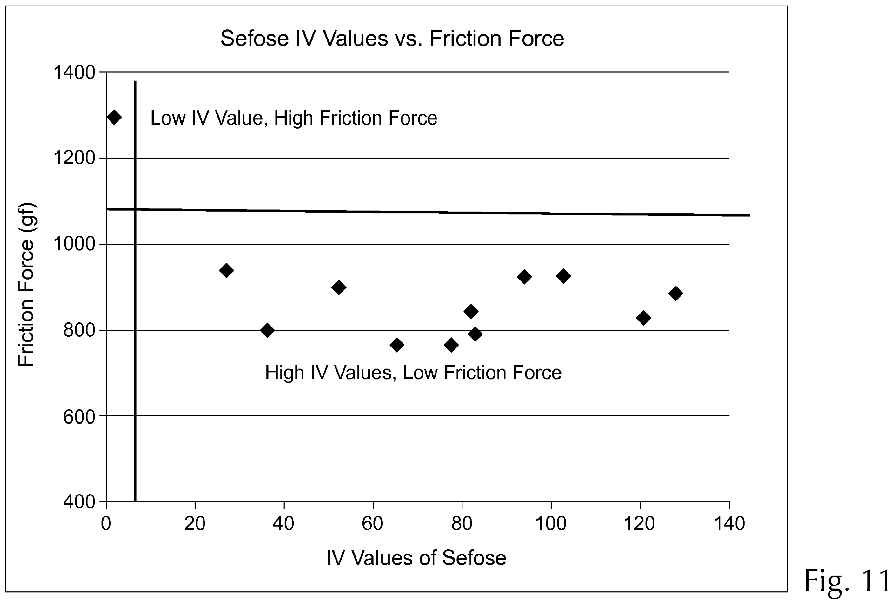

Amongst benefit agents, some contribute to an oily/sticky feel more than others. This is sometimes even seen within a group of benefit agents. For example, the present inventors have surprisingly discovered that when sucrose polyester is a benefit agent, those with an iodine value of 3 or more give a better skin feel. Skin feel can be represented by a measurement called friction force which measures the amount of force exerted by a surface as an object moves across it. A high friction force of about 1100 gf or more would predict an oily and/or sticky feeling on the skin, while a low friction force of about 1050 or less would predict a smooth skin feel. Fig. 11 is a graph showing the relationship of some sucrose polyesters' iodine values with friction force.

In addition to a skin feel benefit, the use of a sucrose polyester with an iodine value of 3 or more also has a more translucent appearance. This is can also drive consumer preference on the shelf

CLEANSING PHASE

The personal care composition of the present invention includes a cleansing phase. The cleansing phase will comprise as least one anionic surfactant. The surfactant may be present from about 3% to about 20%, by weight of the personal care composition. The cleansing phase may contain from 3% to about 20%, from about 5% to about 15%, from about from about 7% to about 15%, from about 5% to about 13%, or any combination of the upper, lower, and included limits within the ranges.

The cleansing phase may be structured. When structured, the cleansing phase is comprised of a structured domain. The structured domain is preferably an opaque structured domain, which is preferably a lamellar phase. The lamellar phase can provide resistance to shear, adequate yield to suspend particles and droplets and at the same time providing long term stability, since it is thermodynamically stable. The lamellar phase tends to have a viscosity that minimizes the need for viscosity modifiers, but they can be included if desired.

Anionic surfactants can be either linear or branched. Examples of some suitable linear anionic surfactants include ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium laureth sulfate, potassium laureth sulfate, sodium lauryl sarcosinate, sodium lauroyl sarcosinate, lauryl sarcosine, cocoyl sarcosine, ammonium cocoyl sulfate, sodium cocoyl isethionate, ammonium lauroyl sulfate, sodium cocoyl sulfate, sodium lauroyl sulfate, potassium cocoyl sulfate, potassium lauryl sulfate, monoethanolamine cocoyl sulfate, sodium tridecyl benzene sulfonate, sodium dodecyl benzene sulfonate, and combinations thereof.

Examples of some suitable branched anionic surfactants include but are not limited to the following surfactants: sodium trideceth sulfate, sodium tridecyl sulfate, sodium $C_{12-13}$ alkyl sulfate, sodium $C_{12-15}$ alkyl sulfate, sodium $C_{11-15}$ alkyl sulfate, sodium $C_{12-18}$ alkyl sulfate, sodium $C_{10-16}$ alkyl sulfate, sodium $C_{12-13}$ pareth sulfate, sodium $C_{12-13}$ pareth-$n$ sulfate, sodium $C_{12-14}$ pareth-$n$ sulfate, and combinations thereof. Other salts of all the aforementioned surfactants are useful, such as TEA, DEA, ammonia, potassium salts. Useful alkoxylates include the ethylene oxide, propylene oxide and EO/PO mixed alkoxylates. Phosphates, carboxylates and sulfonates prepared from branched alcohols are also useful anionic branched surfactants. Branched surfactants can be derived from synthetic alcohols such as the primary alcohols from the liquid hydrocarbons produced by Fischer-Tropsch condensed syngas, for example Safol™ 23 Alcohol available from Sasol North America, Houston, TX; from synthetic alcohols such as Neodol™ 23 Alcohol available from Shell Chemicals, USA; from synthetically made alcohols such as those described in U.S. Patent No. 6,335,312 issued to Coffindaffer, et al on January 1, 2002. Preferred alcohols are Safol™ 23 and Neodol™ 23. Preferred alkoxylated alcohols are Safol™ 23-3 and Neodol™ 23-3. Sulfates can be prepared by conventional processes to high purity from a sulfur based $SO_3$ air stream process, chlorosulfonic acid process, sulfuric acid process, or Oleum process. Preparation via $SO_3$ air stream in a falling film reactor is a preferred sulfation process.

Where the anionic surfactant comprises sodium trideceth (n) sulfate, hereinafter STnS, wherein n defines the average moles of ethoxylation, n can range from 0.5 to 2.7, from 1.1 to 2.5, from 1.8 to 2.2, about 2, or any combination of the end points and included numerals within the ranges. It is understood that a material such as ST2S, for example, may comprise a significant amount of molecules which have no ethoxylate, 1 mole ethoxylate, 3 mole ethoxylate, and so on in a distribution which can be broad, narrow or truncated, still comprising ST2S wherein the average of the distribution is about 2.

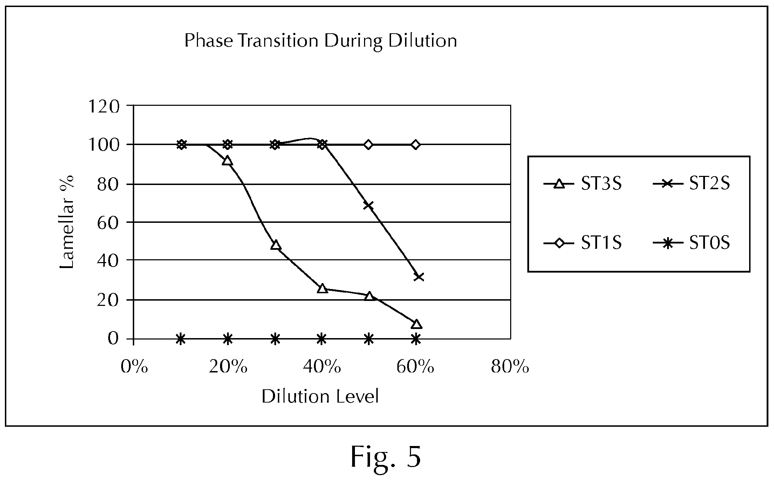

It has been discovered that STnS having fewer than 3 moles of ethoxylation provides surprising structural improvements. Fig. 5 illustrates these improvements by comparing a composition comprising, ST1S, ST2S, and ST3S. At increasing levels of dilution, ST3S begins to transition from a lamellar structure to a micellar structure beginning at about the 19% surfactant level. As such, dilution beyond this level results in a loss of structure. This loss of structure has, until now, necessitated higher concentrations of surfactant to be present within a package. ST2S compositions can remain well structured until a dilution point of 13% surfactant within this example, allowing for the transition to a more micellar structure at much higher dilution levels. ST1S compositions can remain lamellar at even lower surfactant concentrations.

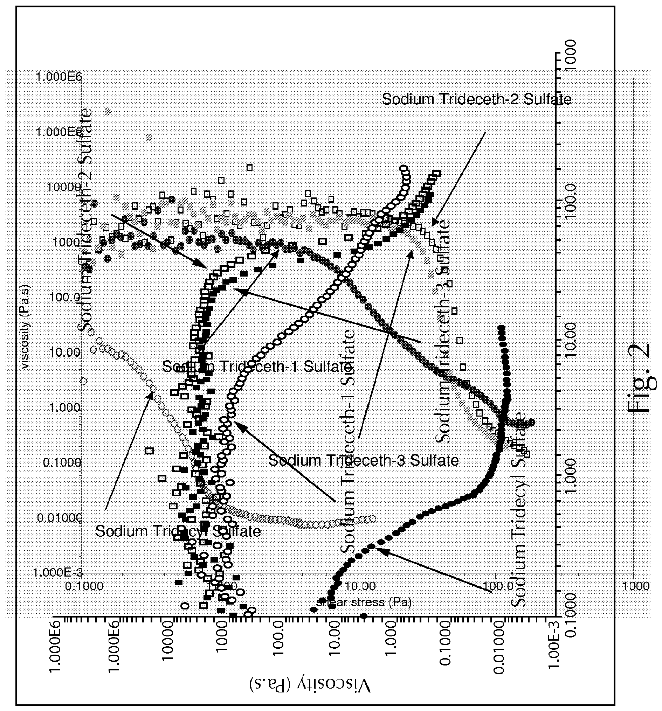

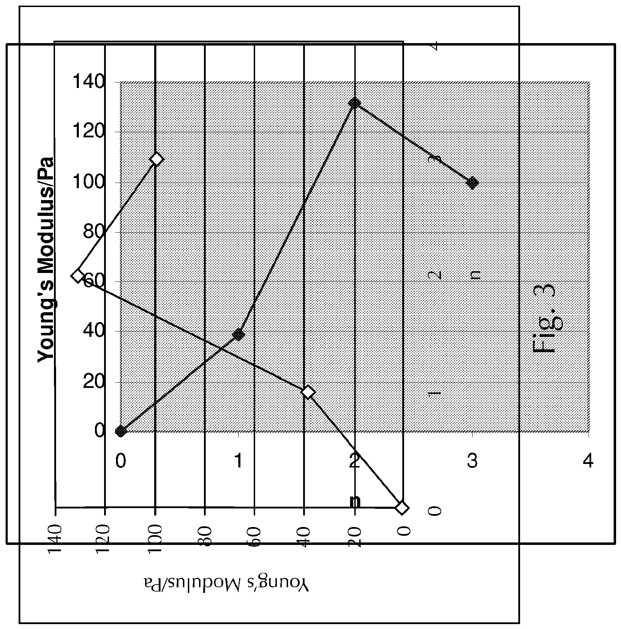

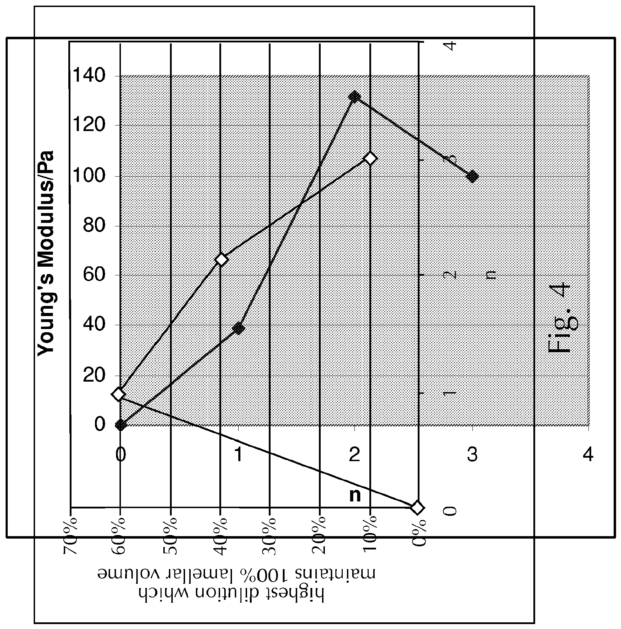

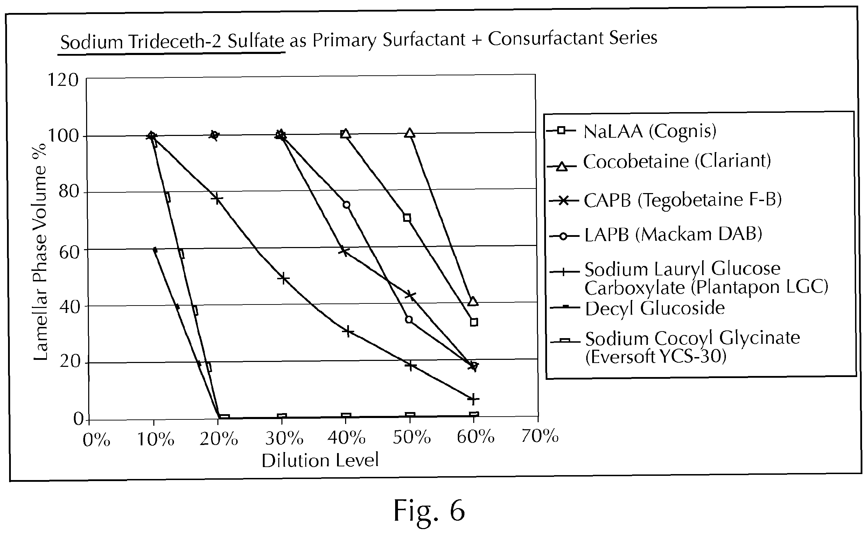

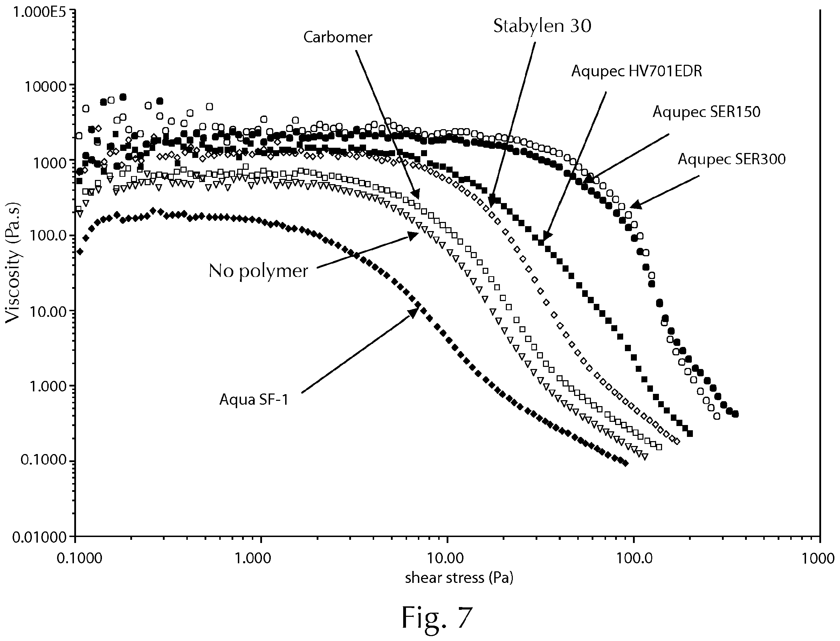

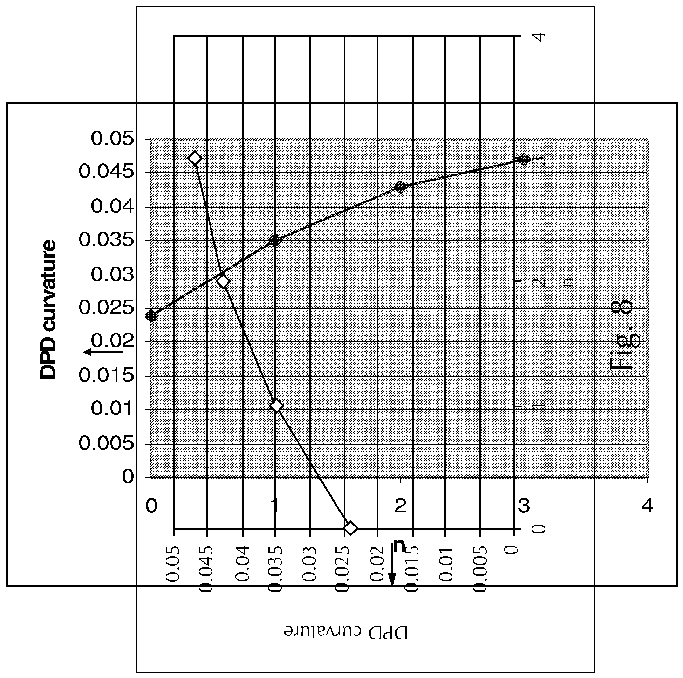

While sodium trideceth sulfate has been disclosed and commercialized, the utilization and benefits of sodium trideceth sulfate having lower ethoxylation values have been unknown, a rationale further supported by the general popularity of ST3S within commercially available products, and the lack of commercial availability of lower ethoxylation products. It is this unknown and surprising result that enables various benefits of the personal care compositions of the present invention, including improved stability, mildness, compatibility, and lather creation. Figs. 2-8 show varying support for this.

Without intending to be limited by theory, the rationale for improved function of STnS, where n is below 3, can be illustrated utilizing dissipative particle dynamics (DPD) simulations. As related to STnS, surfactant aggregates form curved surfaces based on the surfactant shape and interactions between molecules, leading to surfactant architectures which are phases; and to degree of structure of a phase as measured by rheology parameters such as zero shear viscosity. To measure the amount of surfactant curvature, molecular simulations were carried out using DPD by breaking surfactant atoms into beads, where a bead represents typically 3-4 heavy atoms. Simulations were performed in a cube cell with an edge length of approximately 25 nm. The compositions of the simulation boxes varied in average amount of ethoxylation (n = 0 to 3) of STnS. Assembly of surfactants into aggregates starting from random positions was observed during the course of the simulations. DPD Curvature was computed as an average curvature over multiple independent simulations for the surfactant head group-water surface of all resulting objects in a simulation frame, including all bilayers and micelles, and is a relative measure of the average deviation of the colligative surfactant head group surface from flat. DPD Curvature of zero are flat layers with edge defects, which do not form multilamellar vesicles and hence are not expected to exhibit structured rheology, e.g., high zero shear viscosity. At DPD Curvature of about 0.07 and higher, elongated micelle structures are observed to form. At intermediate DPD curvature, curved bilayers can form multilamellar vesicles, leading to high zero shear viscosity and stable compositions.

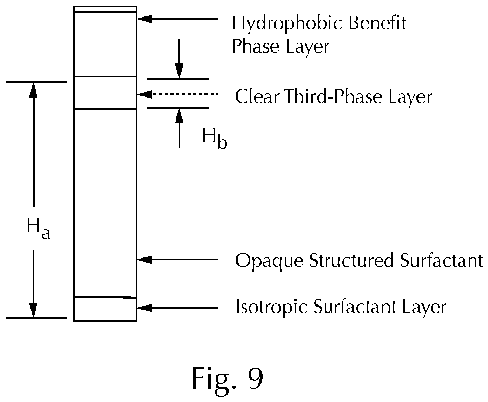

As illustrated in Fig. 9, the simulation results demonstrate bilayers formed from the STnS compositions have lower DPD Curvature of surfactant aggregates with decreasing n. DPD Curvature of ST0S compositions is too low to form compact vesicle structures, whereas the DPD curvature of ST3S compositions is too high so zero shear viscosity is not as high as compared to ST2S compositions of the present invention. Preferred structure is observed for compositions of the present invention having DPD Curvature between about 0.03 and 0.045.

Often, STnS is combined with SLS in order to form a surfactant system. The personal care compositions of the present invention can comprise less than about 5% SLS, less than about 4% SLS, less than about 3% SLS, less than about 2% SLS, less than about 1% SLS, between about 0.1% SLS and about 2% SLS, about 0% SLS. Without wishing to be bound by theory, it is believed that the presence of SLS increases the harshness of the personal care composition, negating at least in part the mildness benefits and/or the efficacy of the benefit agents within the personal care composition.

COSURFACTANT

The personal care compositions of the present invention can further comprise a cosurfactant. Cosurfactants in the present invention comprise from about 0.1% to 20% or from about 2% to about 10%, by weight of the personal care composition. Cosurfactants of the present invention comprise amphoteric surfactants, zwitterionic surfactants, or mixtures thereof.

Amphoteric surfactants suitable for use include those that are broadly described as derivatives of aliphatic secondary and tertiary amines in which the aliphatic radical can be straight or branched chain and wherein one of the aliphatic substituents contains from about 8 to about 18 carbon atoms and one contains an anionic water solubilizing group, e.g., carboxy, sulfonate, sulfate, phosphate, or phosphonate. Examples of compounds falling within this definition are sodium 3-dodecyl-aminopropionate, sodium 3-dodecylaminopropane sulfonate, sodium lauryl sarcosinate, N-alkyltaurines such as the one prepared by reacting dodecylamine with sodium isethionate according to the teaching of U.S. Pat. No. 2,658,072, N-higher alkyl aspartic acids such as those produced according to the teaching of U.S. Pat. No. 2,438,091, and the products described in U.S. Pat. No. 2,528,378. Some more specific examples of suitable amphoteric surfactants comprise lauroamphoacetate, sodium cocoamphoactetate, disodium lauroamphoacetate disodium cocodiamphoacetate, or mixtures thereof. Moreover, amphoacetates and diamphoacetates can also be used.

Zwitterionic surfactants suitable for use include those that are broadly described as derivatives of aliphatic quaternary ammonium, phosphonium, and sulfonium compounds, in which the aliphatic radicals can be straight or branched chain, and wherein one of the aliphatic substituents contains from about 8 to about 18 carbon atoms and one contains an anionic group, e.g., carboxy, sulfonate, sulfate, phosphate, or phosphonate. Zwitterionic surfactants suitable for use in the multiphase, personal care composition include, for example, betaines, including cocoamidopropyl betaine.

STRUCTURING SYSTEM

The personal care composition may also include a structuring system. Most often this system is utilized in the cleansing phase to provide structure to the cleansing phase. A structuring system can include an electrolyte, an associative polymer, and/or a non-ionic emulsifier.

An electrolyte may be present from about 0.5% to about 5%, by weight of the personal care composition. Suitable electrolytes for use herein include, for example, those which comprise an anion selected from the group consisting of phosphate, chloride, sulfate, citrate, and mixtures thereof; and a cation selected from the group consisting of sodium, ammonium, potassium, magnesium, and mixtures thereof. More specific examples of suitable electrolytes include sodium chloride, ammonium chloride, ammonium sulfate, and mixtures thereof.

An associate polymer may be present at a level of about 10% or less, by weigh to of the personal care composition. An example of suitable associative polymers includes Aqupec SER-300 made by Sumitomo Seika of Japan, which is Acrylates/C10-30 alkyl acrylate crosspolymer and comprises stearyl side chains with less than about 1% HM. Other associative polymers comprise stearyl, octyl, decyl and lauryl side chains. Some more exemplary associative polymers are Aqupec SER-150 (acrylates/C10-30 alkyl acrylates crosspolymer) comprising about C18 (stearyl) side chains and about 0.4% HM; Aqupec HV-701EDR which comprises about C8 (octyl) side chains and about 3.5% HM; and Stabylen 30 manufactured by 3V Sigma S.p.A., which has branched isodecanoate hydrophobic associative side chains.

DEPOSITION POLYMERS

The personal care compositions of the present invention can additionally comprise an organic cationic deposition polymer in the one or more phases as a deposition aid for the benefit agents described herein. Suitable cationic deposition polymers for use in the compositions of the present invention contain cationic nitrogen-containing moieties such as quaternary ammonium moieties. Nonlimiting examples of cationic deposition polymers for use in the personal cleansing composition include polysaccharide polymers, such as cationic cellulose derivatives. Preferred cationic cellulose polymers are the salts of hydroxyethyl cellulose reacted with trimethyl ammonium substituted epoxide, referred to in the industry (CTFA) as Polyquaternium 10 which are available from Amerchol Corp. (Edison, N.J., USA) in their Polymer KG, JR and LR series of polymers with the most preferred being KG-30M. Other suitable cationic deposition polymers include cationic guar gum derivatives, such as guar hydroxypropyltrimonium chloride, specific examples of which include the Jaguar series (preferably Jaguar C-17) commercially available from Rhodia Inc., and N-Hance polymer series commercially available from Aqualon.

The deposition polymers of the present invention can have a cationic charge density from about 0.8 meq/g to about 2.0 meq/g or from about 1.0 meq/g to about 1.5 meq/g.

WATER

The surfactant phase of the present invention also comprises water. The surfactant phase of the personal care composition can comprise from about 10% to about 90%, from about 40% to about 85%, from about 60% to about 80%, by weight of the personal care composition of water, or any combination of the upper, lower, and included limits within the ranges.

BENEFIT PHASE

The personal care compositions of the present invention comprise a benefit phase. The benefit phase in the present invention is preferably hydrophobic. The benefit phase can be anhydrous, substantially free of water, or free of water. The benefit phase can be substantially free or free of surfactant.

The benefit phase typically comprises benefit agents. The benefit phase can comprise from about 0.1% to about 50%, preferably from about 1% to about 30%, more preferably from about 5% to about 30%, by weight of the personal care composition, of a benefit agent.

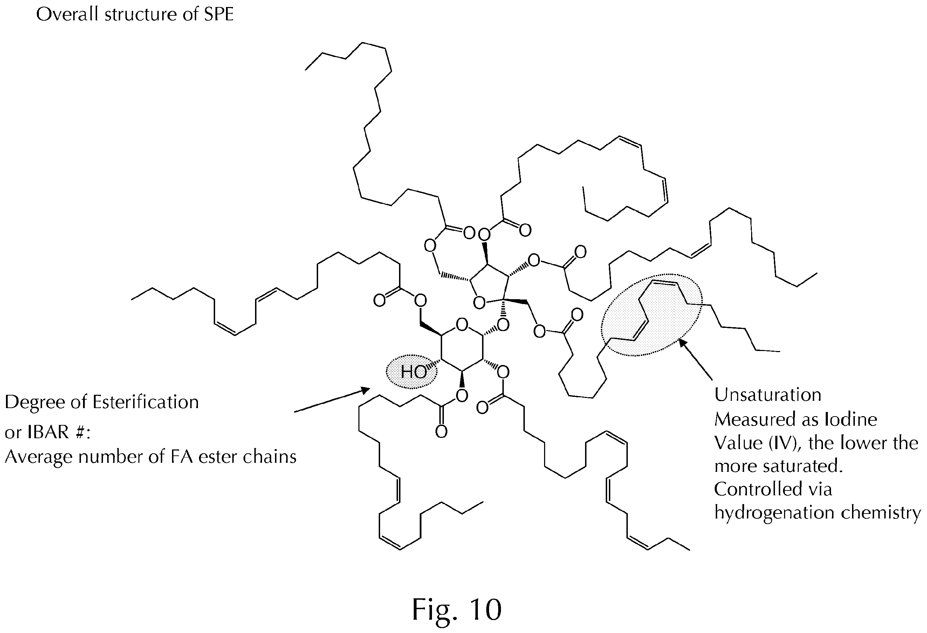

The primary benefit agent that can be included is one or more types of sucrose polyesters. Sucrose polyesters useful herein can include polyester materials, having multiple substitution positions around the sucrose backbone coupled with the chain length, saturation, and derivation variables of the fatty chains. The Sucrose polyesters display both a range of esterification and saturation as shown in Fig. 10.

A sucrose polyester useful as, for example, a benefit agent can have an esterification ("IBAR") of greater than about 5, about 6, about 8, from about 5 to about 8, from about 5 to about 7, or from about 6 to about 8,. As the sucrose polyesters can be derived from a natural resource, a distribution in the IBAR and chain length can exist. For example, a sucrose polyester having an IBAR of 6, can include a mixture that includes mostly an IBAR of about 6 with some IBAR of about 5 and some IBAR of about 7.

Additionally, the sucrose polyester useful as, for example, a benefit agent can have a saturation or iodine value ("IV") of from about 3 to about 140, from about 5 to about 140, from about 10 to about 140, from about 10 to about 130, from about 20 to 100, from about 25 to about 130, or any combination of the upper, lower, and included limits within the ranges. The sucrose polyester can also have a chain length of about C12 to C20.

The benefit agent disclosed herein can include a mixture or blend of sucrose polyesters. For example, the benefit agent can include a mixture or blend of two or more sucrose polyesters. In such a mixture or blend, at least one of the sucrose polyesters can have a melting point greater than about 30° C., an IBAR greater than about 5, an IV of from about 3 to about 70 and at least one of the other sucrose polyesters can have an IBAR of from about 1 to about 8 and an IV of from about 1 and about 135 such that the sucrose polyester mixture or blend has an IBAR of at least 5 and an IV of about 1 and about 135. Additionally, in such a mixture or blend, the ratio of sucrose polyesters having a melting point greater than about 30° C, an IBAR greater than about 5, an IV of from about 3 to about 70 to the sucrose polyesters having an IBAR of from about 1 and about 8, and an IV of from about 1 to about 135 can be about 1:2, about 1:3, about 1:5, about 3:4, and about 3:10. The sucrose polyester mixtures or blends can also have a G' value of from about 0.22 Pa to about 10,030 Pa at 0.01 Hz and a G" value of from about 0.83 Pa to about 23,960 at about 0.01 Hz.

Examples of sucrose polyesters suitable for use herein include, but are not limited to, Sefose 1618S, Sefose 1618U, Sefa Soyate IMF 40, Sefa Soyate LP426, Sefose 1618S B6, Sefose 1618U B6, Sefa Cottonate, all available from The Procter and Gamble Co. of Cincinnati, Ohio. The Sucrose ester and fatty acid distribution of selected Sefose are listed in Fig. 11.

The benefit phase may further contain an additional benefit agent. Suitable additional benefit agents include, for example, glycerides, acetoglyceride esters, alkyl esters, alkenyl esters, polyglycerin fatty acid esters, lanolin, silicone oils, wax esters, glyceryl monooleate, glyceryl monostearate, glyceryl monolaurate, glyceryl dilaurate, petrolatum, mineral oil, or combinations thereof.

Non-limiting examples glycerides suitable for use as benefit agents herein include castor oil, soy bean oil, derivatized soybean oils such as maleated soy bean oil, safflower oil, cotton seed oil, corn oil, walnut oil, peanut oil, olive oil, cod liver oil, almond oil, avocado oil, palm oil and sesame oil, vegetable oils, sunflower seed oil, and vegetable oil derivatives; coconut oil and derivatized coconut oil, cottonseed oil and derivatized cottonseed oil, jojoba oil, cocoa butter, and combinations thereof.

Non-limiting examples of acetoglyceride esters suitable for use as hydrophobic skin benefit agents herein include acetylated monoglycerides.

Non-limiting examples of alkyl esters suitable for use as hydrophobic skin benefit agents herein include isopropyl esters of fatty acids and long chain esters of long chain (i.e. C10-C24) fatty acids, e.g. cetyl ricinoleate, non-limiting examples of which include isopropyl palmitate, isopropyl myristate, cetyl riconoleate and stearyl riconoleate. Other examples are: hexyl laurate, isohexyl laurate, myristyl myristate, isohexyl palmitate, decyl oleate, isodecyl oleate, hexadecyl stearate, decyl stearate, isopropyl isostearate, diisopropyl adipate, diisohexyl adipate, dihexyldecyl adipate, diisopropyl sebacate, acyl isononanoate lauryl lactate, myristyl lactate, cetyl lactate, and combinations thereof.

Non-limiting examples of alkenyl esters suitable for use as hydrophobic skin benefit agents herein include oleyl myristate, oleyl stearate, oleyl oleate, and combinations thereof.

Non-limiting examples of polyglycerin fatty acid esters suitable for use as hydrophobic skin benefit agents herein include decaglyceryl distearate, decaglyceryl diisostearate, decaglyceryl monomyriate, decaglyceryl monolaurate, hexaglyceryl monooleate, glycerol monooleate glycerol monooleate, and combinations thereof.

Non-limiting examples of lanolin and lanolin derivatives suitable for use as hydrophobic skin benefit agents herein include lanolin, lanolin oil, lanolin wax, lanolin alcohols, lanolin fatty acids, isopropyl lanolate, acetylated lanolin, acetylated lanolin alcohols, lanolin alcohol linoleate, lanolin alcohol riconoleate, and combinations thereof.

Non-limiting examples of silicone oils suitable for use as hydrophobic skin benefit agents herein include dimethicone copolyol, dimethylpolysiloxane, diethylpolysiloxane, mixed C1-C30 alkyl polysiloxanes, phenyl dimethicone, dimethiconol, and combinations thereof. Preferred are non-volatile silicones selected from dimethicone, dimethiconol, mixed C1-C30 alkyl polysiloxane, and combinations thereof. Nonlimiting examples of silicone oils useful herein are described in U.S. Patent No. 5,011,681 (Ciotti et al.).

Still other suitable hydrophobic skin benefit agents include milk triglycerides (e.g., hydroxylated milk glyceride) and polyol fatty acid polyesters.

Still other suitable hydrophobic skin benefit agents include wax esters, non-limiting examples of which include beeswax and beeswax derivatives, spermaceti, myristyl myristate, stearyl stearate, and combinations thereof. Also useful are vegetable waxes such as carnauba and candelilla waxes; sterols such as cholesterol, cholesterol fatty acid esters; and phospholipids such as lecithin and derivatives, sphingo lipids, ceramides, glycosphingo lipids, and combinations thereof. Also suitable benefit agents include glycerol monooleate.

The benefit agents for use in the benefit phase can include water insoluble or hydrophobic benefit agents with, for example, a Vaughan Solubility Parameter (VSP) of from about 5 to about 15 or from about 5 to less than 10. These solubility parameters are well known in the formulation arts, and are defined by Vaughan in Cosmetics and Toiletries, Vol. 103, p47-69, Oct. 1988.

ADDITIONAL INGREDIENTS

Additional materials useful in the products herein are categorized or described by their cosmetic and/or therapeutic benefit or their postulated mode of action or function. However, it is to be understood that the active and other materials useful herein can, in some instances, provide more than one cosmetic and/or therapeutic benefit or function or operate via more than one mode of action. Therefore, classifications herein are made for the sake of convenience and are not intended to limit an ingredient to the particularly stated application or applications listed. The precise nature of these additional materials, and levels of incorporation thereof, will depend on the physical form of the composition and the nature of the cleansing operation for which it is to be used.

To further improve stability under stressful conditions such as high temperature and vibration, it is preferable to adjust the densities of the separate phases such that they are substantially equal. To achieve this, low density microspheres can be added to one or more phases of the personal care composition, preferably the structured surfactant phase. Personal care composition that comprises low density microspheres are described in a patent application published on May 13, 2004 under U.S. Patent Publication No. 2004/0092415A1 entitled "Striped Liquid Personal Cleansing Compositions Containing A Cleansing Phase and A Separate Phase with Improved Stability," filed on Oct. 31, 2003 by Focht, et al.

Other non limiting additional ingredients that can be used in the personal care composition of the present invention can comprise a benefit component that is selected from the group consisting of thickening agents; preservatives; antimicrobials; fragrances; chelators (e.g. such as those described in U.S. Pat. No. 5,487,884 issued to Bisset, et al.); sequestrants; vitamins (e.g. Retinol); vitamin derivatives (e.g. tocophenyl actetate, niacinamide, panthenol); sunscreens; desquamation actives (e.g. such as those described in U.S. Pat. No. 5,681,852 and 5,652,228 issued to Bisset); anti-wrinkle/ anti-atrophy actives (e.g. N-acetyl derivatives, thiols, hydroxyl acids, phenol); anti-oxidants (e.g. ascorbic acid derivatives, tocophenol) skin soothing agents/skin healing agents (e.g. panthenoic acid derivatives, aloe vera, allantoin); skin lightening agents (e.g. kojic acid, arbutin, ascorbic acid derivatives) skin tanning agents (e.g. dihydroxyacteone); anti-acne medicaments; essential oils; sensates; pigments; colorants; pearlescent agents; interference pigments (e.g. such as those disclosed in U.S. Pat. No. 6,395,691 issued to Liang Sheng Tsaur, U.S. Pat. No. 6,645,511 issued to Aronson, et al., U.S. Pat. No. 6,759,376 issued to Zhang, et al, U.S. Pat. No. 6,780,826 issued to Zhang, et al.) particles (e.g. talc, kolin, mica, smectite clay, cellulose powder, polysiloxane, silicas, carbonates, titanium dioxide, polyethylene beads) hydrophobically modified non-platelet particles (e.g. hydrophobically modified titanium dioxide and other materials described in a commonly owned, patent application published on Aug. 17, 2006 under Publication No. 2006/0182699A, entitled "Personal Care Compositions Containing Hydrophobically Modified Non-platelet particle filed on Feb. 15, 2005 by Taylor, et al.) and mixtures thereof. In one aspect, the multiphase personal care composition may comprise from about 0.1% to about 4%, by weight of the multiphase personal care composition, of hydrophobically modified titanium dioxide.

Other additional ingredients are most typically those materials approved for use in cosmetics and that are described in the CTFA Cosmetic Ingredient Handbook, Second Edition, The Cosmetic, Toiletries, and Fragrance Association, Inc. 1988, 1992.

TEST METHODS

The current invention utilizes a number of test methods to determine various metrics of structure. The methodology for these tests and associated examples are illustrated below.

Zero Shear Viscosity and Young's Modulus Methods

The Zero Shear Viscosity of a material which is a phase or a composition of the present composition, can be measured either prior to combining in the composition, after preparing a composition, or first separating a phase or component from a composition by suitable physical separation means, such as centrifugation, pipetting, cutting away mechanically, rinsing, filtering, or other separation means.

A controlled stress rheometer such as a TA Instruments AR2000 Rheometer is used to determine the Zero Shear Viscosity. The determination is performed at 25°C with the 4 cm diameter parallel plate measuring system and a 1 mm gap. The geometry has a shear stress factor of 79580 m-3 to convert torque obtained to stress. Serrated plates can be used to obtain consistent results when slip occurs.

First the material is positioned on the rheometer base plate, the measurement geometry (upper plate) is moved into position 1.1 mm above the base plate. Excess material at the geometry edge is removed by scraping after locking the geometry. The geometry is then moved to the target 1 mm position above the base plate and a pause of about 2 minutes is allowed to allow loading stresses to relax. This loading procedure ensures no tangential stresses are loaded at the measurement onset, which can influence results obtained. If the material comprises particles discernible to the eye or by feel (beads, e.g.) which are larger than about 150 microns in number average diameter, the gap setting between the base plate and upper plate is increased to the smaller of 4 mm or 8-fold the diameter of the 95th volume percentile particle diameter. If a phase has any particle larger than 5 mm in any dimension, the particles are removed prior to the measurement.

The measurement is performed by applying a continuous shear stress ramp from 0.1 Pa to 1,000 Pa over a time interval of 4 minutes using a logarithmic progression, i.e., measurement points evenly spaced on a logarithmic scale. Thirty (30) measurement points per decade of stress increase are obtained. If the measurement result is incomplete, for example if material is observed to flow from the gap, results obtained are evaluated with incomplete data points excluded. If there are insufficient points to obtain an accurate measurement, the measurement is repeated with increased number of sample points.

The Young's Modulus (Pa) is obtained by graphing the Stress (Pa) vs. Strain (unitless) and obtaining the slope of the regression line of the initial linear region between Stress vs. Strain, typically occurring in the region below about 4% strain. If the relationship is not linear, the linear regression line slope below 2% strain is taken as the Young's Modulus (Pa), using unitless strain.

The Zero Shear Viscosity is obtained by taking a first median value of viscosity in Pascal-seconds (Pa-sec) for viscosity data obtained between and including 0.1 Pa and the point where viscosity begins to steeply decline. After taking the first median viscosity, all viscosity values greater than 5-fold the first median value and less than 0.2x the median value are excluded, and a second median viscosity value is obtained of the same viscosity data, excluding the indicated data points. The second median viscosity so obtained is the Zero Shear Viscosity.

Compositions of the present invention have a Zero Shear Viscosity of at least about 100 Pa-s, at least about 300 Pa-s, at least about 500 Pa-s, at least about 1000 Pa-s, at least about 1500 Pa-s, or at least about 2000 Pa-s.

Compositions of the present invention have a Young's Modulus of at least about 2 Pa, at least about 5 Pa, at least about 10 Pa, at least about 20 Pa, at least about 30 Pa, at least about 40 Pa, at least about 50 Pa, or at least about 75 Pa.

Ultracentrifugation Method

The Ultracentrifugation Method is a physical method used to determine amount of structure in a composition or a subset of a composition. The method is also used to determine the rate at which a structured surfactant composition dissolves upon dilution to present effective amounts of surfactant to the cleaning environment proximal to surfaces.

A composition is separated by ultracentrifuge into separate but distinguishable layers. The multiphase personal care composition of the present invention can have multiple distinguishable layers (e.g., a structured surfactant layer, and a benefit layer).

First, dispense about 4 grams of composition into a Beckman Centrifuge Tube (11x60mm) to fill the tube. Next, dilute the composition to a 10% Dilution Level using 90% of the composition and 10% DI water using an appropriate mixer and dispense the same amount of composition into a companion centrifuge tube. Continue to dilute the composition and fill tubes in the same manner until a 60% Dilution Level is obtained for the composition using 40% of the composition with 60% DI water. Place the centrifuge tubes in an ultracentrifuge (Beckman Model L8-M or equivalent) using a sling rotor and ultracentrifuge using the following conditions: 50,000 rpm, 2 hours, and 40°C.

Measure the relative phase volumes of the phases the composition by measuring the height of each layer using an Electronic Digital Caliper (within 0.01mm). Layers are identified by those skilled in the art by physical observation techniques paired with chemical identification if needed. For example, the structured surfactant layer is identified by transmission electron microscopically (TEM), polarized light microscopy, and/or X-ray diffraction for the present invention as a structured lamellar phase comprising multilamellar vesicles, and the hydrophobic benefit layer is identified by its low moisture content (less than 10% water as measured by Karl Fischer Titration). The total height $H_a$ is measured which includes all materials in the ultracentrifuge tube. Next, the height of each layer is measured from the bottom of the centrifuge tube to the top of the layer, and the span of each layer algebraically determined by subtraction. The benefit layer may comprise several layers if the benefit phase has more than one component which may phase splits into liquid and waxy layers, or if there is more than one benefit component. If the benefit phase splits, the sum of the benefit layers measured is the benefit layer height, $H_b$,. Generally, a hydrophobic benefit layer when present, is at the top of the centrifuge tube.

The surfactant phase may comprise several layers or a single layer, $H_c$. There may also be a micellar, unstructured, clear isotropic layer at the bottom or next to the bottom of the ultracentrifuge tube. The layers immediately above the isotropic phase generally comprise higher surfactant concentration with higher ordered structures (such as liquid crystals). These structured layers are sometimes opaque to naked eyes, or translucent, or clear. There may be several structured layers present, in which case $H_c$ is the sum of the individual structured layers. If any type of polymer-surfactant phase is present, it is considered a structured phase and included in the measurement of $H_c$. The sum of the aqueous phases is $H_s$.

Finally, the structured domain volume ratio is calculated as follows:

$$\text{Structured Domain Volume Ratio} = H_c / H_s *100\%$$

If there is no benefit phase present, use the total height as the surfactant layer height, $H_s=H_a$. For the present invention, the Structured Domain Volume Ratio is the Lamellar Phase %. The measurement is made for each dilution prepared and centrifuged, i.e., the Structured Domain Volume Ratio is determined for the composition, and for 90%, 80%, 70% and 60% dilutions prepared as indicated above.

The highest amount of dilution (i.e., the lowest Dilution Level) wherein the composition maintains at least 95% Lamellar Phase % is an indicator of amount of structure for compositions having varying n values for STnS.

The highest dilution wherein the composition has at least 95% lamellar phase can be greater than about 15% , greater than about 25%, or greater than about 35%.

The composition can have a Structured Domain Volume Ratio of at least about 40%, at least about 45%, at least about 50%, at least about 55%, at least about 60%, at least about 65%, at least about 70%, at least about 75%, at least about 80%, at least about 85%, and greater than about 90% by volume of the aqueous surfactant composition.

Ultracentrifugation Dilution Method

The Ultracentrifugation Dilution Method is a physical method used to determine amount of structure in a composition at a certain point in its dilution profile, which relates to the ability of the composition to lather. The Ultracentrifugation Dilution Method utilizes the results from the Ultracentrifugation Method at the 50% dilution point. When consumers use surfactant compositions with an implement such as a washcloth or a Puff, about 10 ml of composition is typically dosed onto the implement which can contain about 10 ml of water therein. Consumers agitate to generate lather, requiring the composition to rapidly dissolve at this dilution strength. The ability of structured surfactant compositions to dissolve at 50% Dilution % is measured by the method.

The method is identical in all its details to the Ultracentrifugation Method. The result at 50% Dilution % is obtained for a composition and is expressed as the Diluted 50% Lamellar Phase Volume.

Results from the Ultracentrifugation Dilution Method parallel results obtained for the Dissolution Rate Test for the compositions of the current invention comprising STnS, affirming the relationship between high structure and reduced lather, and vice versa, leading to improved stability and use aesthetics within a narrower range of n values for STnS. The ST0S composition of Example 4 being relatively unstructured, has low structure upon dilution, but is unsuitable for the purposes of a structured surfactant composition due to its inability to provide requisite stabilization to a composition based on its rheology. The ST3S composition of Example 1 has sufficient structure and dilutes rapidly to micellar surfactants useful for lather and cleaning, but disadvantageously these ST3S compositions cannot readily be formulated into compositions comprising reduced surfactant levels; they will always remain costly, inefficient, environmentally less preferred, and less mild. The ST1S composition of Example 3 has a Diluted 50% Lamellar Phase Volume of 100%, which will result in poor lather and cleaning characteristics in many use modes. The ST2S composition of Example 2 demonstrates versatility in that it has a high degree of structure yet dilutes sufficiently to provide a good lather result, the lather performance supported by its Diluted 50% Lamellar Phase Volume value of 70%. ST2S compositions can be prepared at reduced surfactant levels, for example at 15%, or 12%, or 10% or 8% or even 6% surfactant and retain many of the preferred features of the present invention.

The Diluted 50% Lamellar Phase Volume for a personal care composition can be less than about 90%, less than about 80%, or less than 75%.

Dissolution Rate Method

Structured compositions are prone to slow dissolution, hence poor lather characteristics and cleaning can result. Slowly dissolving structured surfactant phases are largely behind the development of the "Puff" implement many years ago, an agitating implement that encourages dissolution, lather and cleaning. Lather and cleaning result from the ability of aqueous surfactant molecules to diffuse to and stabilize air interfaces and soil surfaces. When surfactants remain locked into lamellar or other organized structures, they are unable to diffuse in the aqueous phase and so must first dissolve as individual surfactant monomers and micelles in order to be effective. Dilution and agitation encourage dissolution during use. The Dissolution Rate Method measures the extent of dissolution of a surfactant composition in water.

A straight walled glass beaker is obtained having an inside diameter (i.d.) of 63 mm and an inside height of 87 mm, e.g. Pyrex 250 ml (No. 1000) which are widely available. 150 grams of distilled water at ambient temperature (75°F) is poured into the beaker. A Teflon ® coated magnetic stir bar is added to the beaker. The stir bar is nominally 1.5 inches long x 5/16 inches diameter and octagonally shaped viewed from the end and has a 1/16 in. wide molded pivot ring around its center where the diameter is increased to about 0.35 in. Spinbar ® magnetic stir bars are available from Sigma Aldrich Corp. worldwide including Milwaukee, WI, USA and at www.sigmaaldrich.com.

Measure and record the Initial Water Conductivity of the water using a conductivity meter, e.g., a Mettler-Toledo SevenMulti meter with InLab740 probe, and record the value. The conductivity of the water should be about 2 microSemens/cm (uS/cm) or less to indicate a low level of dissolved solids present. Remove the conductivity probe from the water and place the beaker onto a digitally controlled laboratory stirrer, for example Ika ® Werke RET Control-visc available, e.g., from DivTech Equipment Co, Cincinnati, OH, USA. The beaker is centered on the stirrer and the stirrer is turned on to obtain a constant rotation speed of 500 rpm, establishing a vortex in the water which measures about 3 cm depth from highest point of water at the beaker edge to lowest point of air at the vortex center. Observe the vortex from above to ensure it is centered in the beaker, and the magnetic stir bar centered at the vortex center.

Obtain a cleansing phase and fill it into a 1 ml syringe without entrapping air. The syringe has a diameter of about 1.9 mm at the tip (e.g., BD 1 ml tuberculin slip tip, Becton, Dickinson and Co., Franklin Lakes, NJ, USA). Inject the cleansing phase in a steady stream onto the top surface of the water near the beaker edge but not touching the beaker edge. The composition should be injected in about 1 second. Begin a timer and allow the composition to stir for 30 seconds.

Turn off the stirrer. Insert the conductivity probe into the water in a location away from any undissolved solids. Allow the measurement to stabilize and take a conductivity reading and record the Conductivity.

Turn the stirrer back on. Restart the timer as the digital readout passes 250 rpm. After an additional 30 seconds elapsed time, turn off the stirrer and measure the conductivity in the same manner as previous. Record the Conductivity.

Turn the stirrer back on. Restart the timer as the digital readout passes 250 rpm. After an additional 60 seconds elapsed time, turn off the stirrer and measure the conductivity in the same manner as previous. Record the Conductivity.

Remove the probe from the water without disturbing any remaining solids. Cap the beaker with a suitable watertight cover, e.g., plastic wrap and a rubber band. Shake the beaker vigorously for about 30 seconds to dissolve remaining solids, using a vortex type agitator in addition if necessary.

Uncap the beaker, measure conductivity and record the value as the Final Conductivity. The Dissolution % at each time point is calculated according to the following equation:

$$\text{Dissolution \% } = 100\% \text{ x} \quad \frac{(\text{Conductivity} - \text{Initial Water Conductivity})}{(\text{Final Conductivity} - \text{Initial Water Conductivity})}$$

Repeat the measurement as needed to obtain a representative average value.

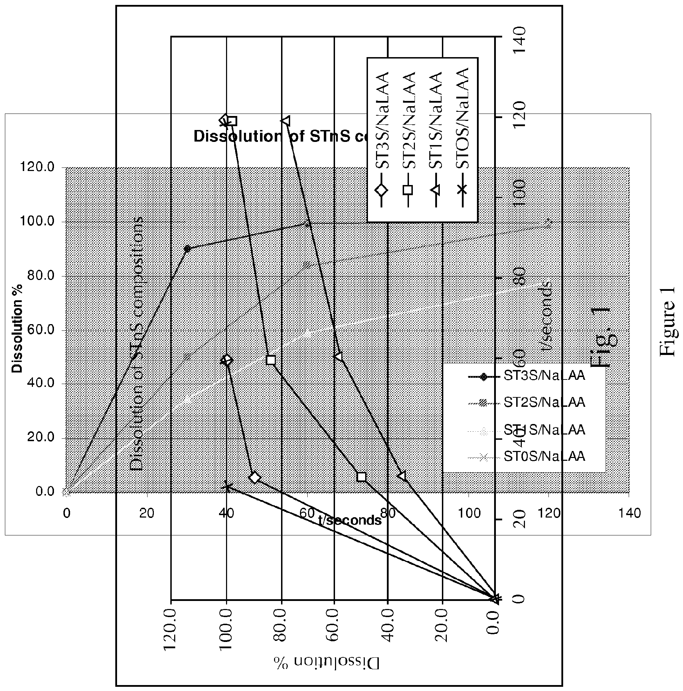

Dissolution testing data on STnS compositions is illustrated in Fig. 1.

At the 60 second time point, compositions of the present invention have a Dissolution % of at least about 60%, at least about 70%, or at least about 80%. At the 120 second time point, compositions of the present invention have a Dissolution % of at least about 80%, at least about 85%, at least about 90%, or at least about 95%.

Third-Phase Method for Determining Structured Surfactant Stability

The "Third-Phase" Method is used to determine structured surfactant phase stability in a personal cleansing composition. The method involves placing the personal care compositions at 50°C for 10days for rapid aging. After rapid aging, transfer about 4 grams of the composition into a Beckman Centrifuge Tube (11x60mm). Place the centrifuge tube in a Beckman LE-80 Ultracentrifuge and operate the Ultracentrifuge under the following conditions: 50,000rpm, 2hours, and @40C.

After Ultracentrifugation, determine the third-phase volume by measuring the height of various surfactant phases using an Electronic Digital Caliper (within 0.01mm) as illustrated in Fig. 10. An example is shown in Fig. 10 for personal cleansing composition comprising Expancel microsphere.

The very top layer is hydrophobic benefit phase layer (hydrocarbons or soybean oil etc.). The layers below the hydrophobic benefit phase layers contain surfactant/water are determined in the following: $H_a$ is the height of all layers containing surfactant/water and $H_b$ is the height of the clear "third-phase" layer just below the hydrophobic benefit phase layer. It is important to record the readings within 30 mins. after the Ultracentrifugation is finished to minimize material migration across different layers. The third phase volume is calculated as: Third-phase Volume% = $H_b/H_a$ *100%

Preferably, the structured surfactant composition comprises less than 10% "third-phase" volume after rapid aging stability protocol. More preferably, the structured surfactant composition comprises less than 5% "third-phase" volume after rapid aging stability protocol. More preferably, the structured surfactant composition comprises less than 2% "third-phase" volume after rapid aging stability protocol. Even more preferably, the structured surfactant composition comprises less than 1% "third-phase" volume after rapid aging protocol. Most preferably, the structured surfactant composition comprises about 0% "third-phase" volume after rapid aging protocol.

EXAMPLES

The following examples describe and demonstrate compositions within the scope of the invention, unless noted otherwise. The examples are given solely for the purpose of illustration and are not to be construed as limitations of the present invention, as many variations thereof are possible without departing from the spirit and scope of the invention. For example, it is contemplated that other compositions, such as hand wash, facial cleanser, and hand dish wash, are also capable of being formulated with this invention.

A surfactant vanilla base composition of Table I (below) can be prepared by first premixing the Aqupec polymer with Trideceth-3. Add water, guar hydroxypropyltrimonium chloride, sodium chloride while mixing. Then, add sodium trideceth-2 sulfate, cocamidopropyl betaine, and the trideceth-3/Aqupec premix. Then add citric to adjust pH to 5.7. Then, add preservatives, and perfume. Keep mixing until homogeneous.

**Table I**

| Surfactant Vanilla Base Compositions (Surfactant VB) | (w/w%) |
|---|---|
| Sodium Trideceth-2 Sulfate | 8.2% |
| Cocamidopropyl betaine | 2.3% |
| Trideceth-3 | 1.0% |
| Guar Hydroxypropyltrimonium chloride | 0.42% |
| Acrylates/C10-C30 alkylacrylates cross polymer (Aqupec SER 300) | 0.2% |

(continued)

| Surfactant Vanilla Base Compositions (Surfactant VB) | (w/w%) |
|---|---|
| Sodium Chloride | 4.75% |
| Citric acid/sodium hydroxide | pH=5.7 |
| Water, perfume, preservatives | Q.S. |

**Table II**

| The following are compositions of Sefose materials with varying degrees of esterification (1 to 8) and I-BAR values. | | | | | | | | | |
|---|---|---|---|---|---|---|---|---|---|
| **Sefose Material** | **SE 1** | **SE 2** | **SE 3** | **SE 4** | **SE 5** | **SE 6** | **SE 7** | **SE 8** | **I-BAR** |
| Sefose 1618U B6 | 0 | 0 | 0 | 3.9 | 17.9 | 14.9 | 39.3 | 24.0 | 6.10 |
| Sefose 1618U | 0 | 0 | 0 | 0 | 0 | 0.0 | 22.0 | 78.0 | 7.76 |
| Sefose 1618S | 0 | 0 | 0 | 0 | 0 | 0.4 | 25.0 | 74.6 | 7.71 |
| Sefose1618S B6 | 0.1 | 0.3 | 1.3 | 8.5 | 22.6 | 27.7 | 26.3 | 14.7 | 5.98 |
| Sefose1618H | 0 | 0 | 0 | 0 | 0.3 | 1.5 | 28.5 | 69.6 | 7.65 |
| Sefa Soyate IMF 40 | 0 | 0 | 0 | 0 | 0.3 | 1.3 | 28.6 | 69.7 | 7.64 |
| Sefa Cottonate | 0 | 0 | 0 | 0 | 0.5 | 1.0 | 22.5 | 76.0 | 7.70 |
| Sefa Soyate LP426 | 0 | 0 | 0 | 0 | 0 | 0 | 25.2 | 74.8 | 7.72 |

**Table III**

| The following are compositions of Sefose materials with varying chain-length distributions, unsaturations, and IV values. | | | | | | | | | | | | |
|---|---|---|---|---|---|---|---|---|---|---|---|---|
| **Sefose Material** | **C14** | **C16** | **C18:0** | **C18:1 t** | **C18:1 c** | **C18:2 t,t** | **C18:2 c,t or t,c** | **C18:2 c,c** | **C18:3** | **C20** | **C22** | **IV** |
| Sefose 1618U B6 | 0.16 | 12.24 | 4.67 | 0.68 | 24.28 | 0 | 0.54 | 50.35 | 6.17 | 0.29 | 0 | 121 |
| Sefose 1618U | 0.1 | 10 | 4 | 0 | 22 | 0 | 0 | 54 | 8 | 0.2 | 0 | 128 |
| Sefose 1618S | 0.13 | 11.94 | 7.8 | 15.52 | 44.15 | 1.16 | 1.54 | 15.49 | 0.93 | 0.31 | 0 | 82 |
| Sefose1618S B6 | 0.25 | 14.53 | 7.01 | 13.11 | 41.76 | 1.19 | 1.68 | 18.65 | 0.65 | 0.27 | 0 | 83 |
| Sefose1618H | 0.16 | 12.43 | 84.77 | 1.05 | 0.88 | 0 | 0 | 0 | 0 | 0.48 | 0 | 2 |
| Sefa Soyate IMF 40 | 0.1 | 11.7 | 42 | 20.4 | 21.8 | 0.7 | 0 | 0 | 0 | 0.3 | 0 | 36 |
| Sefa Cottonate | 0.72 | 22.47 | 3.64 | 5.05 | 27.67 | 0.58 | 2.72 | 35.36 | 1.06 | 0.23 | 0 | 94 |
| Sefa Soyate LP426 | 0 | 10.22 | 12.03 | 36.89 | 38.21 | 1.39 | 0 | 0.5 | 0 | 0.4 | 0.31 | 65 |

**Table IV**

| | | Surfactant Vanilla Base | Lipid Phase | Sefose IV Value |
|---|---|---|---|---|
| The following are examples of personal care compositions comprising a cleansing phase and a lipid phase. The compositions can be prepared by blending the surfactant vanilla base (Surfactant VB) in Table I with the following lipids through SpeedMixing for 2 mins. @ 2,000rpm. | | | | |
| Example 1 | | 95% Surfactant VB | 5% Sefose 1618U B6 | 121 |
| Example 2 | | 95% Surfactant VB | 5% Sefose 1618U | 128 |
| Example 3 | | 95% Surfactant VB | 5% Sefose 1618S | 82 |
| Example 4 | | 95% Surfactant VB | 5% Sefose 1618S B6 | 83 |
| Comparative Example A | | 95% Surfactant VB | 5% Sefose 1618H | 2 |
| Example 5 | | 95% Surfactant VB | 5% Sefa Soyate IMF 40 | 36 |
| Example 6 | | 95% Surfactant VB | 5% Sefose Cottonate | 94 |
| Example 7 | | 95% Surfactant VB | 5% Sefose LP426 | 65 |
| Comparative Example B | | 95% Surfactant VB | 5% Soybean Oil | - |
| Example 8 | | 95% Surfactant VB | 5% Sefose Blend (Sefose 1618H + Sefose 1618U @4:1 Ratio) | 27 |
| Example 9 | | 95% Surfactant VB | 5% Sefose Blend (Sefose 1618H + Sefose 1618U @3:2 Ratio) | 52 |
| Example 10 | | 95% Surfactant VB | 5% Sefose Blend (Sefose 1618H + Sefose 1618U @2:3 Ratio) | 77 |
| Example 11 | | 95% Surfactant VB | 5% Sefose Blend (Sefose 1618H + Sefose 1618U @1:4 Ratio) | 103 |

**Table V**

| | Initial Young's Modulus (Pa) | Final Young's Modulus (Pa) | Lather Volume (ml) | Friction Force (gf) |
|---|---|---|---|---|
| Example 1 | 151 | 22 | 1700 | 829 |
| Example 2 | 28 | 13 | 1750 | 884 |
| Example 3 | 28 | 9.4 | 1750 | 842 |
| Example 4 | 173 | 41 | 2250 | 791 |
| Comparative Example A | 23 | 11 | 2400 | 1294 |
| Example 5 | 42 | 13.5 | 2100 | 799 |
| Example 6 | 24 | 9.2 | 2200 | 925 |
| Example 7 | 28 | 10.3 | 1500 | 765 |
| Comparative Example B | 47 | 7.5 | 2500 | 1235 |
| Example 8 | 24 | 9.2 | 2200 | 925 |
| Example 9 | 28 | 10.3 | 1500 | 765 |

(continued)

| | Initial Young's Modulus (Pa) | Final Young's Modulus (Pa) | Lather Volume (ml) | Friction Force (gf) |
|---|---|---|---|---|
| Example 10 | 26 | 10.8 | 2000 | 898 |
| Example 11 | 23 | 12.9 | 2250 | 938 |

As shown above, Table V illustrates the Young's Modulus, Final Young's Modulus, Lather Volume and Friction Force of the Examples shown in Table IV. Personal care compositions containing Sefoses materials with high IV values had low friction force while the comparative examples containing Sefose with low IV value and Soybean Oil had high friction force. The low fiction force is preferred as it reflects soft/smooth skin feel.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

It should be understood that every maximum numerical limitation given throughout this specification will include every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

All documents cited in the Detailed Description are, in relevant part, incorporated herein by reference; the citation of any document is not to be construed as an admission that it is prior art with respect to the present invention. To the extent that any meaning or definition of a term in this written document conflicts with any meaning or definition of the term in a document incorporated by reference, the meaning or definition assigned to the term in this written document shall govern.

While particular examples of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. A personal care composition comprising a structured cleansing phase and a benefit phase, **characterized by** the structured cleansing phase comprising (a) from 5% to 20%, by weight of said personal care composition, of an anionic surfactant; (b) at least one of an amphoteric surfactant, a zwitterionic surfactant, or a combination thereof; and (c) a structuring system comprising an electrolyte; and the benefit phase comprises from 0.1 % to 50%, by weight of said personal care composition, of a benefit agent comprising a sucrose polyester, wherein the sucrose polyester has an iodine value of 3 or more.

2. The personal care composition of claim 1, wherein the sucrose polyester has an iodine value of 3 to 140, preferably 5 to 140, more preferably 10 to 140, or most preferably 25 to 130.

3. The personal care composition of any preceding claim, wherein the sucrose polyester comprises an esterfication ("IBAR") of greater than or equal to 5, preferably from 5 to 8, more preferably from 6 to 8, or most preferably 6.

4. The personal care composition of any preceding claim, wherein the sucrose polyester comprises a chain length of C12 to C20.

5. The personal care composition of any preceding claim, wherein the benefit agent comprises a blend or mixture of sucrose polyesters.

6. The personal care composition of any preceding claim, wherein said structuring system further comprises from 0.05% to 0.5%, by weight of said personal care composition, of associative polymer.

7. The personal care composition of any preceding claim, wherein said structuring system further comprises a nonionic emulsifier having an HLB from 3.4 to 13.0.

8. The personal care composition of claim 7, wherein said nonionic emulsifier is selected from the group consisting of glyceryl monohydroxystearate, isosteareth-2, trideceth-2, trideceth-3, hydroxystearic acid, propylene glycol stearate, PEG-2 stearate, sorbitan monostearate, glyceryl laurate, laureth-2, cocamide monoethanolamine, lauramide monoethanolamine, and mixtures thereof.

9. The personal care composition of any preceding claim, wherein the electrolyte comprises an anion selected from the group consisting of phosphate, chloride, sulfate, citrate, and mixtures thereof; and a cation selected from the group consisting of sodium, ammonium, potassium, magnesium, and mixtures thereof.

10. The personal care composition of any preceding claim, wherein the anionic surfactant comprises a branched anionic surfactant selected from the group consisting of sodium trideceth (n) sulfate where n is between about 0.5 and about 2.7, sodium trideceth sulfate, sodium tridecyl sulfate, sodium $C_{12-13}$ alkyl sulfate, sodium $C_{12-15}$ alkyl sulfate, sodium $C_{11-15}$ alkyl sulfate, sodium $C_{12-18}$ alkyl sulfate, sodium $C_{10-16}$ alkyl sulfate, sodium $C_{12-13}$ pareth sulfate, sodium $C_{12-13}$ pareth-n sulfate, sodium $C_{12-14}$ pareth-n sulfate, and combinations thereof.

11. The personal care composition of any preceding claim, wherein the anionic surfactant comprises sodium trideceth (n) sulfate, where n is between 0.5 and 2.7, preferably from 1.1 to 2.5, more preferably from 1.8 to 2.2, or most preferably 2.

12. The personal care composition of any preceding claim, wherein said benefit phase comprises less than 10%, more preferably less than 5%, even more preferably less than 3%, even more preferably zero percent, by weight of water.

13. The personal care composition of any preceding claim, wherein said benefit phase is substantially free of surfactant, or more preferably free of surfactant.

**Patentansprüche**

1. Körperpflegezusammensetzung, umfassend eine strukturierte Reinigungsphase und eine Pflegephase, **dadurch gekennzeichnet, dass** die strukturierte Reinigungsphase (a) zu 5 Gew.-% bis 20 Gew.-% der Körperpflegezusammensetzung ein anionisches Tensid; (b) mindestens eines von einem amphoteren Tensid, einem zwitterionischen Tensid, oder einer Kombination davon; und (c) ein Strukturierungssystem, umfassend einen Elektrolyt umfasst; und die Vorteilsphase zu 0,1 % bis 50 Gew.-% der Körperpflegezusammensetzung ein Pflegemittel umfasst, das einen Saccharosepolyester umfasst, wobei der Saccharosepolyester eine Iodzahl von 3 oder mehr aufweist.

2. Körperpflegezusammensetzung nach Anspruch 1, wobei der Saccharosepolyester eine Iodzahl von 3 bis 140, bevorzugt von 5 bis 140, mehr bevorzugt von 10 bis 140, oder am meisten bevorzugt von 25 bis 130 aufweist.

3. Körperpflegezusammensetzung nach einem der vorangehenden Ansprüche, wobei der Saccharosepolyester eine Veresterung ("IBAR") von größer als oder gleich 5, bevorzugt von 5 bis 8, mehr bevorzugt von 6 bis 8 oder am meisten bevorzugt von 6 aufweist.

4. Körperpflegezusammensetzung nach einem der vorangehenden Ansprüche, wobei der Saccharosepolyester eine Kettenlänge von C12 bis C20 aufweist.

5. Körperpflegezusammensetzung nach einem der vorangehenden Ansprüche, wobei das Pflegemittel eine Mischung oder ein Gemisch von Saccharosepolyestern umfasst.

6. Körperpflegezusammensetzung nach einem der vorangehenden Ansprüche, wobei das Strukturierungssystem weiterhin zu 0,05 Gew.-% bis 0,5 Gew.-% der Körperpflegezusammensetzung ein assoziatives Polymer umfasst.

7. Körperpflegezusammensetzung nach einem der vorangehenden Ansprüche, wobei das Strukturierungssystem weiterhin einen nichtionischen Emulgator mit einem HLB-Wert von 3,4 bis 13,0 umfasst.

8. Körperpflegezusammensetzung nach Anspruch 7, wobei der nichtionische Emulgator ausgewählt ist aus der Gruppe bestehend aus Glycerylmonohydroxystearat, Isosteareth-2, Trideceth-2, Trideceth-3, Hydroxystearinsäure, Propylenglycolstearat, PEG-2-stearat, Sorbitanmonostearat, Glyceryllaurat; Laureth-2, Cocamidmonoethanolamin, Lauramidmonoethanolamin und Gemischen davon.

9. Körperpflegezusammensetzung nach einem vorangehenden Anspruch, wobei der Elektrolyt ein Anion, das ausgewählt ist aus der Gruppe bestehend aus Phosphat, Chlorid, Sulfat, Citrat, und Gemischen davon; und ein Kation, das ausgewählt ist aus der Gruppe bestehend aus Natrium, Ammonium, Kalium, Magnesium und Gemischen davon, umfasst.

10. Körperpflegezusammensetzung nach einem der vorangehenden Ansprüche, wobei das anionische Tensid ein verzweigtes anionisches Tensid umfasst, das ausgewählt ist aus der Gruppe bestehend aus Natriumtrideceth-n-sulfat, wobei n zwischen etwa 0,5 und etwa 2,7 beträgt, Natriumtridecethsulfat, Natriumtridecylsulfat, Natrium-$C_{12\text{-}13}$-alkylsulfat, Natrium-$C_{12\text{-}15}$-alkylsulfat, Natrium-$C_{11\text{-}15}$-alkylsulfat, Natrium-$C_{12\text{-}18}$-alkylsulfat, Natrium-$C_{10\text{-}16}$-alkylsulfat, Natrium-$C_{12\text{-}13}$-parethsulfat, Natrium-$C_{12\text{-}13}$-pareth-n-sulfat, Natrium-$C_{12\text{-}14}$-pareth-n-sulfat, und Kombinationen davon.

11. Körperpflegezusammensetzung nach einem der vorangehenden Ansprüche, wobei das anionische Tensid Natriumtrideceth-n-sulfat umfasst, wobei n zwischen 0,5 und 2,7, bevorzugt von 1,1 bis 2,5, mehr bevorzugt von 1,8 bis 2,2, oder am meisten bevorzugt 2 beträgt.

12. Körperpflegezusammensetzung nach einem der vorangehenden Ansprüche, wobei die Pflegephase weniger als 10 %, mehr bevorzugt weniger als 5 %, noch mehr bevorzugt weniger als 3 %, noch mehr bevorzugt Null Gew.-% Wasser umfasst.

13. Körperpflegezusammensetzung nach einem der vorangehenden Ansprüche, wobei die Pflegephase im Wesentlichen von Tensid frei ist, oder mehr bevorzugt von Tensid frei ist.

**Revendications**

1. Composition de soin personnel comprenant une phase nettoyante structurée et une phase bénéfique, **caractérisée par** la phase nettoyante structurée comprenant (a) de 5 % à 20 % en poids de ladite composition de soin personnel, d'un agent tensioactif anionique ; (b) au moins l'un parmi un agent tensioactif amphotère, un agent tensioactif zwittérionique, ou une combinaison de ceux-ci ; et (c) un système structurant comprenant un électrolyte ; et la phase bénéfique comprend de 0,1 % à 50 % en poids de ladite composition de soin personnel, d'un agent bénéfique comprenant un polyester de saccharose, dans laquelle le polyester de saccharose a un indice d'iode de 3 ou plus.

2. Composition de soin personnel selon la revendication 1, dans laquelle le polyester de saccharose a un indice d'iode de 3 à 140, de préférence 5 à 140, plus préférablement 10 à 140, ou le plus préférablement 25 à 130.

3. Composition de soin personnel selon une quelconque revendication précédente, dans laquelle le polyester de saccharose comprend une estérification (« IBAR ») supérieure ou égale à 5, de préférence de 5 à 8, plus préférablement de 6 à 8, ou le plus préférablement 6.

4. Composition de soin personnel selon une quelconque revendication précédente, dans laquelle le polyester de saccharose comprend une longueur de chaîne de C12 à C20.

5. Composition de soin personnel selon une quelconque revendication précédente, dans laquelle l'agent bénéfique comprend un mélange ou une mixture de polyesters de saccharose.

6. Composition de soin personnel selon une quelconque revendication précédente, dans laquelle ledit système structurant comprend en outre de 0,05 % à 0,5 % en poids de ladite composition de soin personnel, de polymère associatif.

7. Composition de soin personnel selon une quelconque revendication précédente, dans laquelle ledit système structurant comprend en outre un émulsifiant non ionique ayant un rapport hydrophile-lipophile de 3,4 à 13,0.

8. Composition de soin personnel selon la revendication 7, dans laquelle ledit émulsifiant non ionique est choisi dans le groupe constitué de monohydroxystéarate de glycéryle, isostéareth-2, tridéceth-2, tridéceth-3, acide hydroxystéarique, stéarate de propylène glycol, stéarate de PEG-2, monostéarate de sorbitan, laurate de glycéryle, laureth-2, monoéthanolamine d'amide de coprah, monoéthanolamine de lauramide, et des mélanges de ceux-ci.

9. Composition de soin personnel selon une quelconque revendication précédente, dans laquelle l'électrolyte comprend un anion choisi dans le groupe constitué de phosphate, chlorure, sulfate, citrate et des mélanges de ceux-ci ; et un cation choisi dans le groupe constitué de sodium, ammonium, potassium, magnésium et des mélanges de ceux-ci.

10. Composition de soin personnel selon une quelconque revendication précédente, dans laquelle l'agent tensioactif anionique comprend un agent tensioactif anionique ramifié choisi dans le groupe constitué de tridéceth (n) sulfate de sodium où n est compris entre environ 0,5 et environ 2,7, tridéceth sulfate de sodium , tridécyl sulfate de sodium, alkylsulfate en $C_{12}$ à $_{13}$ de sodium, alkylsulfate en $C_{12}$ à $_{15}$ de sodium, alkylsulfate en $C_{11}$ à $_{15}$ de sodium, alkylsulfate en $C_{12}$ à $_{18}$ de sodium, alkylsulfate en $C_{10}$ à $_{16}$ de sodium, pareth-sulfate en $C_{12}$ à $_{13}$ de sodium, pareth-n-sulfate en $C_{12}$ à $_{13}$ de sodium, pareth-n-sulfate en $C_{12}$ à $_{14}$ de sodium, et des combinaisons de ceux-ci.

11. Composition de soin personnel selon une quelconque revendication précédente, dans laquelle l'agent tensioactif anionique comprend du tridéceth (n) sulfate de sodium, où n est compris entre 0,5 et 2,7, de préférence de 1,1 à 2,5, plus préférablement de 1,8 à 2,2, ou le plus préférablement 2.

12. Composition de soin personnel selon une quelconque revendication précédente, dans laquelle ladite phase bénéfique comprend moins de 10 %, plus préférablement moins de 5 %, encore plus préférablement moins de 3 %, encore plus préférablement zéro pour cent, en poids d'eau.

13. Composition de soin personnel selon une quelconque revendication précédente, dans laquelle ladite phase bénéfique est essentiellement exempte d'agent tensioactif, ou plus préférablement exempte d'agent tensioactif.

Figure 1

Fig. 2

Fig. 3

Fig. 4

Fig. 5

EP 2 718 413 B1

Sodium Trideceth-2 Sulfate as Primary Surfactant + Consurfactant Series

Fig. 6

Fig. 7

Fig. 8

Fig. 9

Overall structure of SPE

Degree of Esterification
or IBAR #:
Average number of FA ester chains

Unsaturation
Measured as Iodine
Value (IV), the lower the
more saturated.
Controlled via
hydrogenation chemistry

Fig. 10

EP 2 718 413 B1

Fig. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040223991 A1
- US 6335312 B
- US 2658072 A
- US 2438091 A
- US 2528378 A
- US 5011681 A, Ciotti
- US 20040092415 A1
- US 5487884 A, Bisset

- US 5681852 A
- US 5652228 A
- US 6395691 B, Liang Sheng Tsaur
- US 6645511 B, Aronson
- US 6759376 B, Zhang
- US 6780826 B, Zhang
- US 20060182699 A

**Non-patent literature cited in the description**

- **VAUGHAN.** *Cosmetics and Toiletries,* October 1988, vol. 103, 47-69

- CTFA Cosmetic Ingredient Handbook. The Cosmetic, Toiletries, and Fragrance Association, Inc, 1988